Europäisches Patentamt

European Patent Office

Office européen des brevets

(19)

(11) Publication number: **0 080 759**
**B1**

# (12) EUROPEAN PATENT SPECIFICATION

(45) Date of publication of patent specification: **07.08.85**

(51) Int. Cl.⁴: **C 07 C 39/07, C 07 C 37/07**

(21) Application number: **82201464.3**

(22) Date of filing: **18.11.82**

(54) **Process for the preparation of 3,5-dimethylphenol.**

(30) Priority: **02.12.81 GB 8136372**

(43) Date of publication of application:
**08.06.83 Bulletin 83/23**

(45) Publication of the grant of the patent:
**07.08.85 Bulletin 85/32**

(84) Designated Contracting States:
**BE CH DE FR GB IT LI NL**

(56) References cited:
**GB-A-1 197 802**
**GB-A-1 197 803**
**GB-A-1 197 804**
**GB-A-1 451 570**

(73) Proprietor: **SHELL INTERNATIONALE
RESEARCH MAATSCHAPPIJ B.V.
Carel van Bylandtlaan 30
NL-2596 HR Den Haag (NL)**

(72) Inventor: **Van Seters, Antonius J. C.
Badhuisweg 3
NL-1031 CM Amsterdam (NL)**
Inventor: **Wattimena, Freddy
Badhuisweg 3
NL-1031 CM Amsterdam (NL)**

(74) Representative: **Aalbers, Onno et al
P.O. Box 302
NL-2501 CH The Hague (NL)**

Courier Press, Leamington Spa, England.

**0 080 759**

**Description**

This invention relates to a process for the preparation of 3,5-dimethylphenol (3,5-xylenol or 1,3-dimethyl-5-hydroxybenzene) by converting isophorone (3,5,5-trimethyl-2-cyclohexen-1-one) in th. gasphase in the presence of a catalyst.

The main requirements of a heterogeneous catalyst for this process are that it should give high conversions of the isophorone with a high selectivity towards the desired dimethylphenol. At the same time it should have a reasonable mechanical strength under the process conditions and should not give rise to the formation of large amounts of carbon, which would necessitate frequent regeneration of the catalyst. Also it is desirable to employ high space velocities and just moderately high temperatures.

Various catalysts have been proposed for the process, and of these an attractive catalyst with which satisfactory conversions have been obtained in commercial operation is the catalyst described in UK Patent Specification No. 1,451,570, i.e. a catalyst comprising cobalt and molybdenum on an aluminium-containing carrier, which catalyst has been calcined at a temperature above 800°C. A higher selectivity than is obtained with the catalyst described in Specification No. 1,451,570 has been an implicit need in the art not only for the advantage of increased yield but also for the processing reason that the 3,5-dimethylphenol product cannot be separated easily from the isophorone starting material.

It is the object of the present invention to provide a new catalytic process for the preparation of 3,5-dimethylphenol from isophorone in which a higher conversion can be attained at acceptable selectivity, space velocity and temperature requirements of the process.

According to the present invention a process for the preparation of 3,5-dimethylphenol by converting isophorone in the gas phase in the presence of a catalyst is characterized in that the catalyst employed comprises one or more catalytically active rare earth metals on an alumina-based carrier. In order to achieve commercially attractive selectivities said catalyst should normally have a specific area not exceeding an area of the order of 1.0 $m^2/g$, and preferred catalysts in this respect will have a specific area below 0.5 $m^2/g$.

The rare earth metals comprise the elements having atomic numbers 21, 39 and 57 to 71 inclusive. These elements often occur together, and are difficult to separate. Investigation has shown that the presence on the alumina-based carrier of a rare earth metal having little or no catalytic activity for the conversion of isophorone to 3,5-dimethylphenol is not in practice detrimental to the efficacy of the catalyst. Consequently, there is no practical reason to effect an unnecessary difficult separation of rare earth metals, and providing, of course, a given mixture thereof has catalytic activity for this conversion, which can be determined readily by routine experiment, such mixtures of rare earth metals can be used, without isolating, or even without determining, the element or elements in any particular catalytically active mixture actually provide(s) the catalytic activity. It has been found that naturally occurring mixtures of rare earth metals appear to provide at least one which is catalytically active. Such mixtures, in particular didymium, are therefore eligable for use in carrying out the present invention. Although classically the expression "didymium", "Di", represented a mixture of neodymium and praseodymium, it is now commonly used to designate all the lanthanons other than cerium in their natural ratio of abundance. (cf. Kirk-Othmer, Encyclopedia of Chemical Technology (1968), Vol. 17, p. 147).

The carrier is based on alumina, $Al_2O_3$, and whilst it can contain minor amounts of other known carrier materials such as silica or magnesia, preferably pure alumina is used and in particular alpha or gamma-alumina. The required specific area of the catalyst, which is preferably below 0.5 $m^2/g$, can be achieved either by selection of an appropriate grade of alumina carrier or by calcination of the impregnated carrier at temperatures of the order of 1100°C to achieve a surface area reduction in the manner well-known in principle to catalyst experts.

The amount of rare earth metal — or compound thereof calculated as metal — is not very critical, and, in fact, may be limited by the absorption capacity of the carrier. Suitably the catalyst contains 0.5 to 20% by weight of rare earth metal based on the weight of the carrier, preferably not more than 8% by weight; and usually between 2 and 4% by weight will be sufficient.

The rare earth catalyst may be promoted with several other metals or metal compounds. The catalyst can also contain up to 4%, based on the weight of the carrier, of an alkali namely lithium, sodium, potassium, rubidium and cesium, or an alkaline earth metal, namely magnesium, calcium, strontium and barium, or a mixture thereof.

In particular the amount of alkali or alkaline earth metal is from 0.01 to 1.0% by weight, especially from 0.1 to 0.4% by weight, based on the carrier. Excellent results have been obtained when potassium is present.

Either in addition to, or instead of alkali or alkaline earth metal, the catalyst may contain a promoting amount of a transition metal, in particular up to 4% based on the weight of the carrier of a transition metal selected from Groups IB, VIB and VIII of the Periodic System. These transition metals are, respectively, copper, silver and gold; chromium, molybdenum and tungsten; and iron, cobalt, nickel, ruthenium, rhodium, palladium, osmium, iridium and platinum. Preferably the amount of transition metal is from 0.01 to 1.0%, especially 0.1 to 0.4% by weight, based on the carrier. Preferably, the transition metal is copper, silver, cobalt and/or molybdenum, especially cobalt.

It is found that the addition of the promotors brings about a further improvement, in particular in the

2

selectivity of the catalyst. Most preferably both cobalt and potassium are present as promotors.

The metals may be introduced onto the carrier by any convenient method, for example by impregnation of the carrier with aqueous solutions of soluble salts of the metals. The metal or metals is (are) present in the final catalyst in oxide form, the salt(s)-impregnated carrier being dried and calcined after treatment with an aqueous salt(s) solution, for example, of metal nitrates or acetates. The calcination can be effected at a temperature in the range from 450 to 550°C or higher up to 625°C depending on the temperature at which it will be used in the process of the invention. This can be followed by calcination at a much higher temperature, for example, 1100°C if a surface reduction is also required.

The process of the invention is conveniently carried out a temperature of 525 to 625°C, which range lies about 25°C below the range specified for the catalyst according to UK Patent Specification No. 1,451,570. Whilst this represents an energy saving, another important advantage is a lower carbon deposit ratio. The lifetime of the catalyst, i.e. the period between regenerations, is increased substantially thereby. The pressure used may be atmospheric pressure or slightly above atmospheric pressure. The isophorone may also be mixed with a suitable diluent, for example, hydrogen or steam which may help further to reduce carbon formation. The space velocities employed in the process according to the invention may also be higher by a factor of 2 to 5 than in the process according to UK Patent Specification No. 1,451,570. A preferred weight hourly space velocity is from 0.5 to 1.0 kg isophorone/litre catalyst/hour.

The immediate reaction product of the process of the invention is predominantly 3,5-dimethylphenol containing minor amounts of by-products such as toluene, xylene, mesitylene, m-cresol, dihydro-isophorone and trimethylphenol. These by-products can be removed from the main product by known procedures, for example, by the methods described in UK Patent Specification No. 1,197,802. By employing a catalyst of specific area not exceeding about 1.0 m$^2$/g so as to avoid a degree of "dead" (open) space between catalyst particles in which side reactions occurring therein would lead to unacceptable by-product formation, attractive selectivities can be attained when carrying out the process of the invention. This has the dual advantage for the process, of achieving higher yields and facilitating work-up procedures as compared to the known processes.

3,5-Dimethylphenol is used in the preparation of xylenol-formaldehyde resins. After chlorination to 4-chloro- and 2,4-dichloro-3,5-dimethylphenol it is used as a disinfectant and a wood preservative. It is also a starting material for the total synthesis of vitamin E, as well as for other compounds e.g. dyes, agricultural, pharmaceutical and aroma chemicals.

The invention is further illustrated in the following Examples.

Examples I—VII

The following Examples show the effect of varying rare earth metal content on the catalyst performance.

The catalysts containing didymium oxide, Di$_2$O$_3$ (composition: 45.8%w La$_2$O$_3$, 9.5%w Pr$_6$O$_{11}$, 32.5%w Nd$_2$O$_3$, 5.5%w Sm$_2$O$_3$, 3.5%w Gd$_2$O$_3$, 0.5%w Y$_2$O$_3$, 2.7%w other rare earth oxides) were prepared by impregnating a commercially available alpha-alumina carrier material (specific area 0.3 m$^2$/g; pore volume 0.2 ml/g; particle size 0.25—0.60 mm) with a solution of didymium nitrate in water, followed by drying at 120°C for ½ hour and calcination at 500°C for four hours.

A sample of the catalyst (14.2 g; 12 ml) was placed in a microflow reactor. Pre-heated isophorone of purity ca 97% was passed over the catalyst at a temperature of 600°C, a pressure of 1 bar absolute, and varying space velocities. The conversion of isophorone and the selectivity towards 3,5-dimethylphenol were determined by gas-liquid chromatography and are expressed in molar percentages. All data were obtained after 50 ml of isophorone had been brought into the reactor.

For comparative purposes a catalyst without rare earths (Comparative Experiment A) and a catalyst according to British patent specification 1,451,570 (Comp. Exp. B, C) were tested under the same conditions. Also the thermal conversion, i.e. in the absence of any catalyst, was measured (Comp. Exp. D—G).

The results are shown in Table I.

## 0 080 759

TABLE I

| Example | Catalyst | WHSV (kg/l. h) | Conversion (%) | Selectivity (%) |
|---|---|---|---|---|
| I | 8.6% Di/α Al$_2$O$_3$ | 0.20 | 93 | 80 |
| II | id. | 0.50 | 83 | 82 |
| III | id. | 1.00 | 68 | 84 |
| IV | 4% Di/α Al$_2$O$_3$ | 0.25 | 93 | 79 |
| V | id | 0.50 | 80 | 79 |
| VI | 2% Di/α Al$_2$O$_3$ | 0.25 | 90 | 79 |
| VII | id. | 0.50 | 74 | 75 |
| A | α Al$_2$O$_3$ | 0.30 | 40 | 68 |
| B | 4.2% CoO/10.8% MoO$_3$/ 0.48% K on γ-Al$_2$O$_3$ | 0.17 | 91 | 79 |
| C | id. | 0.33 | 79 | 75 |
| D | none | 0.07* | 82 | 60 |
| E | id. | 0.14* | 70 | 61 |
| F | id. | 0.35* | 50 | 54 |
| G | id. | 0.70* | 34 | 46 |

*kg isophorone/l of empty reactor space at 590—610°C/hour.

### Examples VIII—XXI

These Examples show the effect of promotion with transition metals. The preparation of the catalysts was as in the preceding Example, except that the Al$_2$O$_3$-carrier material was impregnated with an aqueous solution of a transition metal nitrate as well. The reaction conditions were again 600°C and 1 bar. The results are tabulated in Table II.

4

# 0 080 759

TABLE II

| Example | Catalyst | WHSV (kg/l. h) | Conversion (%) | Selectivity (%) |
|---------|----------|----------------|----------------|-----------------|
| VIII | 8% Di/0.8% Mo | 0.25 | 95 | 81 |
| IX | id. | 0.50 | 82 | 79 |
| X | id. | 1.00 | 76 | 80 |
| XI | 8% Di/0.4% Mo | 0.26 | 77 | 77 |
| XII | id. | 0.53 | 76 | 77 |
| XIII | 8% Di/0.8% Co | 0.25 | 100 | 82 |
| XIV | id. | 0.45 | 99 | 80 |
| XV | id. | 0.92 | 85 | 85 |
| XVI | 4% Di/0.4% Cu | 0.25 | 93 | 80 |
| XVII | id. | 0.50 | 73 | 70 |
| XVIII | 4% Di/0.4% Ag | 0.25 | 94 | 70 |
| XIX | id. | 0.50 | 74 | 72 |
| XX | 4% Di/0.4% Co | 0.185 | 98 | 78 |
| XXI | id. | 0.46 | 98 | 75 |

### Examples XXII—XXIII

These Examples show the effect of promotion with alkali metal. The preparation of the catalyst was as in the first seven Examples, except that the $Al_2O_3$-carrier material was impregnated with an aqueous solution of potassium nitrate as well. The reaction conditions were again 600°C and 1 bar. The results are tabulated in Table III.

TABLE III

| Example | Catalyst | WHSV (kg/l. h) | Conversion (%) | Selectivity (%) |
|---------|----------|----------------|----------------|-----------------|
| XXII | 4% Di/0.4% K | 0.50 | 100 | 80 |
| XXIII | id. | 1.00 | 95 | 79 |

### Examples XXIV—XXXII

These Examples show the effect of promotion with both cobalt and potassium. The preparation of the catalysts was analogous to the preceding Examples. The reaction conditions were again 600°C and 1 bar. The results are shown in Table IV.

5

TABLE IV

| Example | Catalyst | WHSV (kg/l. h) | Conversion (%) | Selectivity (%) |
|---------|----------|----------------|----------------|-----------------|
| XXIV | 4% Di/0.1% Co/0.4% K | 0.50 | 100 | 85 |
| XXV | id. | 1.00 | 95 | 85 |
| XXVI | 0.4% Co/0.4% K | 0.50 | 99 | 80 |
| XXVII | id. | 1.00 | 97 | 80 |
| XXVIII | 0.4% Co/0.8% K | 0.50 | 100 | 79 |
| XXIX | id. | 1.00 | 95 | 81 |
| XXX | 0.4% Co/0.1% K | 0.50 | 100 | 81 |
| XXXI | 0.1% Co/0.1% K | 0.50 | 99 | 85 |
| XXXII | id. | 1.00 | 98 | 83 |

It is apparent from the above Examples that the best results are obtained using a catalyst comprising 4.0% Di/0.1% Co/0.1 to 0.4% K on $\alpha$ $Al_2O_3$.

## Examples XXXIII—XXXVI

These Examples show the effect of different temperatures on the preferred catalysts. The results are shown in Table V which also includes the results obtained in Examples XXX and XXXI and Comparative Experiments B, H and J, the latter referring to the catalyst described in British patent specification No. 1,451,570.

TABLE V

| Example | Catalyst | WHSV (kg/l. h) | Conversion (%) | Selectivity (%) | Temperature (°C) |
|---------|----------|----------------|----------------|-----------------|------------------|
| XXX | see Table IV | 0.50 | 100 | 81 | 600 |
| XXXIII | id. | 0.50 | 61 | 82 | 550 |
| XXXIV | id. | 0.25 | 94 | 82 | 550 |
| XXXI | see Table IV | 0.50 | 99 | 85 | 600 |
| XXXV | id. | 0.50 | 71 | 82 | 550 |
| XXXVI | id. | 0.67 | 99 | 80 | 630 |
| B | see Table I | 0.17 | 91 | 79 | 600 |
| H | id. | 0.25 | 62 | 77 | 550 |
| J | id. | 0.25 | 23 | 60 | 500 |

## Claims

1. Process for the preparation of 3,5-dimethylphenol by converting isophorone in the gas phase in the presence of a catalyst, characterized in that the catalyst employed comprises one or more catalytically active rare earth metals on an alumina-based carrier.

2. A process as claimed in claim 1, characterized in that said catalyst has a specific area not exceeding an area of the order of 1.0 m²/g.

3. A process as claimed in claim 1, characterized in that said catalyst has a specific area not exceeding 0.5 m²/g.

4. A process as claimed in any one of claims 1 to 3, characterized in that the catalyst contains a naturally occurring mixture of rare earth metals.

5. A process as claimed in any one of claims 1 to 4, characterized in that the alumina-based carrier consists of alpha alumina.

6. A process as claimed in any one of claims 1 to 5, characterized in that the catalyst contains 0.15 to 20% of rare earth metal based on the weight of the carrier.

7. A process as claimed in any one of claims 1 to 6, characterized in that the catalyst also contains up to 4% based on the weight of the carrier of an alkali and/or alkaline earth metal.

8. A process as claimed in claim 7, characterized in that the amount of alkali and/or alkaline earth metal is from 0.01 to 1.0% by weight.

9. A process as claimed in claim 7 or 8, characterized in that the alkali metal is potassium.

10. A process as claimed in any one of claims 1 to 9, characterized in that the catalyst also contains up to 4% based on the weight of the carrier of a transition metal selected from Groups IB, VIB and VIII of the Periodic System.

11. A process as claimed in claim 10, characterized in that the amount of transition metal is from 0.01 to 1.0% by weight.

12. A process as claimed in claim 10 or 11, characterized in that the transition metal is copper, silver and/or molybdenum.

13. A process as claimed in claim 10 or 11, characterized in that the transition metal is cobalt.

14. A process as claimed in any one of claims 1 to 12, characterized in that the process is carried out at a temperature of 525 to 625°C.

15. A process as claimed in any one of claims 1 to 14, characterized in that the process is carried out at a weight hourly space velocity of 0.5 to 1.0 kg isophorone/l catalyst/hour.

16. Catalytic composition for the preparation of 3,5-dimethylphenol by conversion in the gas phase of isophorone, comprising an alumina-based carrier containing 0.5 to 10% of catalytically active rare earth metal(s), 0 to 4% of alkali(ne earth) metal(s) and 0 to 4% of transition metal(s) selected from Groups IB, VIB and VIII of the Periodic System, based on the weight of the carrier, the catalyst having a specific surface not exceeding an area of the order of 1.0 m²/g.


**Patentansprüche**

1. Verfahren zur Herstellung von 3,5-Dimethylphenol durch Umwandlung von isophoron in der Gasphase in Gegenwart eines Katalysators, dadurch gekennzeichnet, daß der angewandte Katalysator ein oder mehrere katalytischwirksame seltene Erdmetalle und einen Träger auf Tonerdebasis umfaßt.

2. Verfahren nach Anspruch 1, dadurch gekennzeichnet, daß der Katalysator eine spezifische Oberfläche in der Größenordnung von nicht mehr als 1,0 m²/g besitzt.

3. Verfahren nach Anspruch 1, dadurch gekennzeichnet, daß der Katalysator eine spezifische Oberfläche von nicht mehr als 0,5 m²/g besitzt.

4. Verfahren nach einem der Ansprüche 1 bis 3, dadurch gekennzeichnet, daß der Katalysator ein natürlich vorkommendes Gemisch seltener Erdmetalle enthält.

5. Verfahren nach einem der Ansprüche 1 bis 4, dadurch gekennzeichnet, daß der Träger auf Tonerdebasis aus α-Tonerde besteht.

6. Verfahren nach einem der Ansprüche 1 bis 5, dadurch gekennzeichnet, daß der Katalysator 0,15 bis 20% seltene Erdmetalle, bezogen auf das Gewicht des Trägers, enthält.

7. Verfahren nach einem der Ansprüche 1 bis 6, dadurch gekennzeichnet, daß der Katalysator außerdem bis zu 4%, bezogen auf das Gewicht des Trägers, eines Alkali- und/oder Erdalkalimetalls enthält.

8. Verfahren nach Anspruch 7, dadurch gekennzeichnet, daß die Menge an Alkali- und/oder Erdalkalimetall 0,01 bis 1 Gew.-% beträgt.

9. Verfahren nach Anspruch 7 oder 8, dadurch gekennzeichnet, daß das Alkalimetall Kalium ist.

10. Verfahren nach einem der Ansprüche 1 bis 9, dadurch gekennzeichnet, daß der Katalysator außerdem bis zu 4%, bezogen auf das Gewicht des Trägers, eines Übergangsmetalls, ausgewählt aus den Gruppen IB, VIB und VIII des Periodensystems, enthält.

11. Verfahren nach Anspruch 10, dadurch gekennzeichnet, daß die Menge an Übergangsmetall 0,01 bis 1,0 Gew.-% beträgt.

12. Verfahren nach Anspruch 10 oder 11, dadurch gekennzeichnet, daß das Übergangsmetall Kupfer, Silber und/oder Molybdän ist.

13. Verfahren nach Anspruch 10 oder 11, dadurch gekennzeichnet, daß das Übergangsmetall Cobalt ist.

14. Verfahren nach einem der Ansprüche 1 bis 12, dadurch gekennzeichnet, daß das Verfahren bei einer Temperatur von 525 bis 625°C durchgeführt wird.

15. Verfahren nach einem der Ansprüche 1 bis 14, dadurch gekennzeichnet, daß das Verfahren bei einem gewichtsmäßigen Durchsatz von 0,5 bis 1,0 kg Isophoron/l Katalysator.h durchgeführt wird.

7

16. Katalytisches Mittel zur Herstellung von 3,5-Dimethylphenol durch Umwandlung von Isophoron in der Gasphase, umfassend einen Träger auf Tonerdebasis, enthaltend 0,5 bis 10% katalytisch wirksames seltenes Erdmetall bzw. seltene Erdmetalle, 0 bis 4% Alkali (Erdalkali)metall(e) und 0 bis 4% Übergangsmetall(e), ausgewählt aus den Gruppen IB, VIB und VIII des Periodensystems, bezogen auf das Gewicht des Trägers, wobei der Katalysator eine spezifische Oberfläche von nicht mehr als etwa 1,0 m²/g besitzt.

**Revendications**

1. Procédé de préparation de 3,5-diméthylphénol par transformation d'isophorone dans la phase gazeuse en présence d'un catalyseur, caractérisé en ce que le catalyseur utilisé comprend un ou plusieurs métaux de terres rares catalytiquement actifs sur un support à base d'alumine.

2. Un procédé selon la revendication 1, caractérisé en ce que le catalyseur a une surface spécifique ne dépassant pas une valeur de l'ordre de 1,0 m²/g.

3. Un procédé selon la revendication 1, caractérisé en ce que le catalyseur a une surface spécifique ne dépassant pas 0,5 m²/g.

4. Un procédé selon l'une quelconque des revendications 1 à 3, caractérisé en ce que le catalyseur contient un mélange de métaux de terres rares existant dans la nature.

5. Un procédé selon l'une quelconque des revendications 1 à 4, caractérisé en ce que le support à base d'alumine est constitué d'alpha-alumine.

6. Un procédé selon l'une quelconque des revendications 1 à 5, caractérisé en ce que le catalyseur contient de 0,15 à 20% de métal de terre rare par rapport au poids du support.

7. Un procédé selon l'une quelconque des revendications 1 à 6, caractérisé en ce que le catalyseur contient aussi jusqu'à 4%, par rapport au poids du support, d'un métal alcalin et/ou alcalino-terreux.

8. Un procédé selon la revendication 7, caractérisé en ce que la quantité de métal alcalin et/ou alcalino-terreux est comprise entre 0,01 et 1,0% en poids.

9. Un procédé selon la revendication 7 ou 8, caractérisé en ce que le métal alcalin est du potassium.

10. Un procédé selon l'une quelconque des revendications 1 à 9, caractérisé en ce que le catalyseur contient aussi jusqu'à 4%, par rapport au poids du support, d'un métal de transition choisi dans les groupes IB, VIB et VIII de la classification périodique des éléments.

11. Un procédé selon la revendication 10, caractérisé en ce que la quantité de métal de transition est comprise entre 0,01 et 1,0% en poids.

12. Un procédé selon la revendication 10 ou 11, caractérisé en ce que le métal de transition est du cuivre, de l'argent et/ou du molybdène.

13. Un procédé selon la revendication 10 ou 11, caractérisé en ce que le métal de transition est du cobalt.

14. Un procédé selon l'une quelconque des revendications 1 à 12, caractérisé en ce que le procédé est mis en oeuvre à une température de 525 à 625°C.

15. Un procédé selon l'une quelconque des revendications 1 à 14, caractérisé en ce que le procédé est mis en oeuvre à une vitesse spatiale horaire en poids de 0,5 à 1,0 kg d'isophorone par litre de catalyseur et par heure.

16. Composition catalytique pour la préparation de 3,5-diméthylphénol par transformation dans la phase gazeuse d'isophorone, comprenant un support à base d'alumine contenant 0,5 à 10% d'un ou plusieurs métaux de terres rares catalytiquement actifs, 0 à 4% d'un ou plusieurs métaux alcalins ou alcalino-terreux et 0 à 4% d'un ou plusieurs métaux de transition choisis dans les groupes IB, VIB et VIII de la classification périodique des éléments, par rapport au poids du support, le catalyseur ayant une surface spécifique ne dépassant pas une valeur de l'ordre de 1,0 m²/g.